# EUROPÄISCHE PATENTANMELDUNG

(11) **EP 2 033 668 A2**
(43) Veröffentlichungstag der Anmeldung: **11.03.2009**
(21) Anmeldenummer: 08160328.4
(22) Anmeldetag: 14.07.2008
(51) Int. Cl.: A61L 31/02, A61L 31/10

(54) **Implantat aus einer biokorrodierbaren Magnesiumlegierung und mit einer Beschichtung aus einem biokorrodierbaren Polyphosphazen**

(30) Priorität: 17.08.2007 DE 102007038799
(71) Anmelder: Biotronik VI Patent AG, 6341 Baar (CH)
(72) Erfinder: Adden, Nina, 90427, Nürnberg (DE)
(74) Vertreter: Lindner-Vogt, Karin L.

(57) **Zusammenfassung**

Die Erfindung betrifft ein Implantat aus einem biokorrodierbaren metallischen Werkstoff und mit einer Beschichtung bestehend aus oder enthaltend ein biokorrodierbares Polyphosphazen.

## Beschreibung

### Technisches Gebiet

Die Erfindung betrifft ein Implantat aus einer biokorrodierbaren Magnesiumlegierung, das eine Beschichtung aufweist.

### Hintergrund der Erfindung und Stand der Technik

Implantate haben in vielfältiger Ausführungsform Anwendung in der modernen Medizintechnik gefunden. Sie dienen beispielsweise der Unterstützung von Gefäßen, Hohlorganen und Gangsystemen (Endovaskuläre Implantate), zur Befestigung und temporären Fixierung von Gewebeimplantaten und Gewebstransplantationen, aber auch zu orthopädischen Zwecken, zum Beispiel als Nagel, Platte oder Schraube.

So hat sich zum Beispiel die Implantation von Stents als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die tragende Umfangswandung wird in der Regel von einer gitterartigen Tragstruktur gebildet, die es erlaubt, den Stent in einem komprimierten Zustand mit kleinem Außendurchmesser bis zur behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Um unnötige Gefäßbeschädigungen zu vermeiden, sollte der Stent nach dem Aufweiten und nach Entfernen des Ballons nicht oder nur in geringem Umfang elastisch zurückfedern, so dass der Stent beim Aufweiten nur wenig über den gewünschten Enddurchmesser hinaus geweitet werden muss. Weitere Kriterien, die in Bezug auf einen Stent wünschenswert sind, umfassen beispielsweise eine gleichmäßige Flächenabdeckung und eine Struktur, die eine gewisse Flexibilität in Bezug auf die Längsachse des Stents erlaubt. In der Praxis wird zur Realisation der genannten mechanischen Eigenschaften der Stent in der Regel aus einem metallischen Werkstoff geformt.

Neben den mechanischen Eigenschaften eines Stents sollte dieser aus einem biokompatiblen Material bestehen, um Abstoßungsreaktionen vorzubeugen. Derzeit werden bei etwa 70% aller perkutanen Interventionen Stents eingesetzt, in 25% aller Fälle kommt es jedoch zu einer In-Stent Restenose aufgrund eines überschießenden neointimalen Wachstums, das durch eine starke Proliferation der arteriellen glatten Muskelzellen und eine chronische Entzündungsreaktion hervorgerufen wird. Zur Senkung der Restenoseraten werden verschiedene Lösungsansätze verfolgt.

Ein Ansatz zur Minderung der Restenoserate sieht vor, auf dem Stent eine pharmazeutisch aktive Substanz (Wirkstoff) bereitzustellen, die den Mechanismen der Restenose entgegenwirkt und den Heilungsverlauf unterstützt. Der Wirkstoff wird in Reinform oder eingebettet in eine Trägermatrix als Beschichtung aufgetragen oder in Kavitäten des Implantats gefüllt. Beispiele umfassen die Wirkstoffe Sirolimus und Paclitaxel.

Ein weiterer, aussichtsreicher Ansatz zur Lösung des Problems liegt in der Verwendung biokorrodierbarer Metalle und deren Legierungen, denn zumeist ist eine dauerhafte Stützfunktion durch den Stent nicht erforderlich; das zunächst geschädigte Körpergewebe regeneriert. So wird beispielsweise in DE 197 31 021 A1 vorgeschlagen, medizinische Implantate aus einem metallischen Werkstoff zu formen, dessen Hauptbestandteil Eisen, Zink oder Aluminium sind bzw. ein Element aus der Gruppe der Alkalimetalle oder Erdalkalimetalle. Als besonders geeignet werden Legierungen auf Basis von Magnesium, Eisen und Zink beschrieben. Nebenbestandteile der Legierungen können Mangan, Kobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest < 1% bekannt, die sich insbesondere zur Herstellung einer Endoprothese, z. B. in Form eines selbstexpandierenden oder ballonexpandierbaren Stents, eignet. Der Einsatz von biokorrodierbaren metallischen Werkstoffen in Implantaten dürfte zu einer deutlichen Minderung von Abstoßungs- oder Entzündungsreaktionen führen.

Die Kombination aus Wirkstofffreisetzung und biokorrodierbarem metallischen Werkstoff erscheint besonders aussichtsreich. Der Wirkstoff wird als Beschichtung aufgebracht oder in eine Kavität im Implantat eingebracht - zumeist eingebettet in eine Trägermatrix. Bekannt sind beispielsweise Stents aus einer biokorrodierbaren Magnesiumlegierung mit einer Beschichtung aus einem Poly(L-lactid). Es hat sich jedoch gezeigt, dass der Abbau bekannter polymerer Beschichtungen bei Stents aus einer biokorrodierbaren Magnesiumlegierung beschleunigt ist. Dies kann unter anderem auf die stark basischen Bedingungen zurückgeführt werden, die sich in Folge des Abbaus der Magnesiumlegierung einstellen. Ferner können die häufig sauren Produkte des Abbaus der polymeren Beschichtung zu einer inflammatorischen Reaktion des umgebenden Gewebes führen, d.h. das Material zeigt nur eine mäßige Biokompatibilität.

### Zusammenfassung der Erfindung

Der Erfindung liegt die Aufgabe zugrunde, ein oder mehrere der geschilderten Probleme zu mindern oder zu überwinden.

Ein erster Aspekt der Erfindung liegt in der Bereitstellung eines Implantats aus einer biokorrodierbaren Magnesiumlegierung und mit einer Beschichtung bestehend aus oder enthaltend ein biokorrodierbares Polyphosphazen.

Polyphosphazene sind Polymere der allgemeinen Struktur der Formel (1), die ein Polymer-Rückgrat aufweisen, das alternierend aus Phosphor- und Stickstoff-Atomen aufgebaut ist. Die beiden verbleibenden Valenzen am Phosphor entsprechen den Substituenten R:

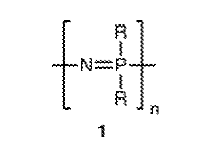

Bei biokorrodierbaren Polyphosphazenen steht R vorzugsweise für einen Substituenten, der durch Kupplung mit einem primären oder sekundären Amin oder einem Aminosäureester entsteht. Zur Steuerung der Abbaugeschwindigkeit kann R neben den genannten Substituenten auch eine Alkoxygruppe sein. Die genannten Polyphosphazene werden vorzugsweise durch Reaktion von Polydichlorphosphazen mit dem gewünschten Amin oder Aminosäureester hergestellt (analog oder entsprechend Adv. Drug Del. Rev. 2003, 55, 467; Biotech. Bioeng. 1996, 52, 102; J Biomed Mater Res 2007, 80A, 661).

Nach einer bevorzugten Ausführungsform ist R ein Substituent, der durch Kupplung mit einem α-Aminosäureester der allgemeinen Formel (2)

H₂NCHR'COOR" (2)

entsteht. R' steht dabei für einen kanonischen oder nicht-kanonischen Rest einer proteinogenen Aminosäure. R" ist ein Alkylrest mit 1 - 10 C-Atomen, vorzugsweise Methyl oder Ethyl. Hierdurch kann die Abbaugeschwindigkeit des Polymers in einfacher Weise beeinflusst werden und der Abbau des Polymers führt zu naturidentischen Produkten, die sehr geringe oder gar keine Nebenwirkungen zeigen. Größere hydrophobere Gruppen R' und R" führen zu einem langsameren Abbau des Polyphosphazens. Besonders bevorzugt ist R ein Substituent, der durch Kupplung mit einem Methyl- oder Ethylester der Aminosäuren Glycin, Alanin, Valin oder Phenylalanin entsteht.

Die Abbaugeschwindigkeit kann auch dadurch verringert werden, dass der Substituent R in geringem Umfang durch einen mäßig oder nicht biokorrodierbaren Substituenten ersetzt wird, z.B. durch Umsatz des Vorprodukts Polydichlorphosphazen mit geringen Mengen an Methylamin oder Ethanol.

Das Polyphosphazen hat ein Molekulargewicht zwischen 10.000 und 10.000.000 g/mol, vorzugsweise zwischen 100.000 und 5.000.000 g/mol.

Die Abbaugeschwindigkeit des Polyphosphazens liegt zwischen 3 und 600 Tagen, bevorzugt zwischen 20 und 360 Tagen. Diese wird nicht beeinflusst durch die basischen Bedingungen, die durch den Abbau des degradierbaren metallischen Werkstoffes entstehen.

Eine Beschichtung im Sinne der Erfindung ist eine zumindest abschnittsweise Auftragung der Komponenten auf den Grundkörper des Implantats, insbesondere Stents. Vorzugsweise wird die gesamte Oberfläche des Grundkörpers des Implantats/Stents von der Beschichtung bedeckt. Eine Schichtdicke liegt vorzugsweise im Bereich von 1 nm bis 100 µm, besonders bevorzugt 300 nm bis 20 µm. Die Beschichtung besteht aus einem biokorrodierbaren Polyphosphazen oder sie enthält ein solches Polyphosphazen. Der Gewichtsanteil von Polyphosphazen an den die Trägermatrix bildenden Komponenten der Beschichtung beträgt mindestens 30%, vorzugsweise mindestens 50%, besonders bevorzugt mindestens 70%. Es kann ein Blend aus verschiedenen Polyphosphazenen vorliegen. Die Komponenten der Beschichtung umfassen die als Trägermatrix agierenden Materialien, also Materialien die für die funktionellen Eigenschaften der Trägermatrix notwendig sind, z. B. auch Hilfsstoffe zur Verbesserung der Viskositätseigenschaften, Gelbildung und Verarbeitbarkeit. Diese Komponenten umfassen nicht die gegebenenfalls zugesetzten Wirkstoffe oder Markermaterialien. Die Beschichtung wird direkt auf die Implantatoberfläche aufgebracht oder es wird erst eine Haftvermittler-Schicht eingesetzt. Diese können z.B. auf die Oberfläche des Grundmaterials aufgebrachte Silane oder Phosphonate mit einer reaktiven Endgruppe (COOH, OH, NH₂, Aldehyd) oder eine oxidische Konversionsschicht des Grundmaterials sein.

Die erfindungsgemäß eingesetzten Polyphosphazene sind hochgradig biokompatibel und biokorrodierbar. Die Verarbeitung kann nach Standardverfahren für die Beschichtung erfolgen. Es können einschichtige, aber auch mehrschichtige Systeme (z.B. so genannte base coat, drug coat oder top coat - Schichten) erstellt werden.

Das Polyphosphazen kann als Trägermatrix für pharmazeutische Wirkstoffe, insbesondere anti-proliferative Wirkstoffe wie zum Beispiel Sirolimus, Everolimus, Biolimus und Paclitaxel sowie anti-inflammatorische Wirkstoffe wie Pimecrolimus, und/oder für Markermaterial wie beispielsweise Röntgenmarker, vorzugsweise Wolframcarbid oder feindisperses Gold, und/oder Magnetresonanzmarker agieren. Der Röntgenmarker kann bei Implantaten aus einem biokorrodierbaren metallischen Werkstoff nicht direkt auf das Produkt aufgebracht werden, da er z. B. die Degradation des Stents durch Bildung von Lokalelementen beeinflussen würde. In der Matrix aus Polyphosphazen ist der Marker dagegen vom Grundkörper abgeschirmt.
Als biokorrodierbar im Sinne der Erfindung werden metallische oder polymere Werkstoffe bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert.

Unter einer biokorrodierbare Magnesiumlegierung im Sinne der Erfindung wird ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Vorzugsweise enthält die biokorrodierbare Magnesiumlegierung Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet. Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

Das Polyphosphazen und die Magnesiumlegierung sind so in ihrer Zusammensetzung zu wählen, dass sie biokorrodierbar sind. Als Prüfmedium zur Testung des Korrosionsverhaltens von polymeren Werkstoffen oder Legierungen dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/I). Eine Probe des zu untersuchenden Werkstoffs wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren.

Vorzugsweise ist das Implantat ein Stent. Stents herkömmlicher Bauart weisen eine filigrane Stützstruktur aus metallischen Streben auf, die zur Einbringung in den Körper zunächst in einem nicht-expandierten Zustand vorliegt und die am Ort der Applikation dann in einen expandierten Zustand aufgeweitet wird. Aufgrund der Verwendungsweise sind spröde Beschichtungssysteme ungeeignet; Polyphosphazene haben dagegen besonders geeignete Materialeigenschaften, wie eine für die Zwecke hinreichende Viskosität und Flexibilität. Der Stent kann vor oder nach dem Crimpen auf einen Ballon beschichtet werden.

Ein zweiter Aspekt der Erfindung bezieht sich auf die Verwendung von biokorrodierbaren Polyphosphazenen als Beschichtungsmaterial für einen Stent aus einer biokorrodierbaren Magnesiumlegierung.

Die Erfindung wird nachfolgend anhand eines Ausführungsbeispiels näher erläutert.

### Ausführungsbeispiel 1: Beschichtung von absorbierbaren Metallstents

Ein Stent aus der biokorrodierbaren Magnesiumlegierung WE43 (97 Gew.% Magnesium, 4 Gew.% Yttrium, 3 Gew.% Seltenerdmetalle außer Yttrium) wird wie folgt beschichtet:

Es wird eine Lösung eines Polyphosphazens in Tetrahydrofuran angesetzt (30 Gew.%). Das eingesetzte Polyphosphazen hat Phenylalaninethylester und Ethylglycinat Seitengruppen im Verhältnis 1,4 : 0,6. Die Synthese ist von Carampin et al beschrieben (J Biomed Mater Res, 2007, 80A, 661). Dieser Lösung kann nach Bedarf ein Medikament zugesetzt werden.

Der Stent wird von Staub und Rückständen gereinigt und in eine geeignete Stentbeschichtungsapparatur (DES Coater, Eigenentwicklung Fa. Biotronik) eingespannt. Mit Hilfe eines Airbrush Systems (Fa. EFD oder Spraying System) wird der sich drehende Stent unter konstanten Umgebungsbedingungen (Raumtemperatur; 42% Luftfeuchte) halbseitig beschichtet. Bei einem Düsenabstand von 20 mm ist ein 18 mm langer Stent nach ca. 10 min beschichtet. Nach Erreichen der beabsichtigten Schichtmasse wird der Stent 5 min bei RT getrocknet, ehe nach Drehen des Stents und erneutem Einspannen die unbeschichtete Seite auf dieselbe Weise beschichtet wird. Der fertig beschichtete Stent wird für 36 h bei 40°C in einem Vakuumofen (Vakucell; Fa. MMM) getrocknet.

Eine Schichtdicke des aufgetragenen Polyphosphazens beträgt etwa 15 µm.

### Ausführungsbeispiel 2: Beschichtung von absorbierbaren Metallstents

Ein Stent aus der biokorrodierbaren Magnesiumlegierung WE43 (97 Gew.% Magnesium, 4 Gew.% Yttrium, 3 Gew.% Seltenerdmetalle außer Yttrium) wird wie folgt beschichtet:

Es wird eine Lösung eines Polyphosphazens in Chloroform angesetzt (5 Gew.%). Das eingesetzte Polyphosphazen hat Phenylalaninethylester und Glycinethylester Seitengruppen im Verhältnis 0,5 : 1,5. Die Synthese erfolgt analog zu Carampin et al (J Biomed Mater Res, 2007, 80A, 661). Dieser Lösung kann nach Bedarf ein Medikament zugesetzt werden.

Der Stent wird von Staub und Rückständen gereinigt und an einem Haken befestigt. Mit Hilfe eines Dipping Systems (Fa. Specialty Coating Systems) wird der Stent unter konstanten Umgebungsbedingungen (Raumtemperatur; 42% Luftfeuchte) in die Lösung getaucht und mit einer Geschwindigkeit von 1 mm pro Minute wieder herausgezogen. Der Stent wird 5 min bei RT getrocknet: Mehrere Tauch-Durchgänge sind möglich. Der fertig beschichtete Stent wird für 36 h bei 40°C in einem Vakuumofen (Vakucell; Fa. MMM) getrocknet.

Eine Schichtdicke des aufgetragenen Polyphosphazens beträgt etwa 20 µm.

## Patentansprüche

1. Implantat aus einer biokorrodierbaren Magnesiumlegierung und mit einer Beschichtung bestehend aus oder enthaltend ein biokorrodierbares Polyphosphazen.

2. Implantat nach Anspruch 1, bei dem das Implantat ein Stent ist.

3. Implantat nach Anspruch 1 oder 2, bei dem die Beschichtung einen Wirkstoff oder/und ein Markermaterial enthält.

4. Implantat nach einem der vorhergehenden Ansprüche, bei dem das Polyphosphazene ein Polymer der Formel (1) 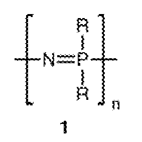 ist und R für einen Substituenten steht, der durch Kupplung mit einem primären oder sekundären Amin oder einem Aminosäureester entsteht.

5. Implantat nach Anspruch 4, bei dem R ein Substituent ist, der durch Kupplung mit einem α-Aminosäureester der allgemeinen Formel (2)
H₂NCHR'COOR" (2)
entsteht, wobei R' für einen kanonischen oder nicht-kanonischen Rest einer proteinogenen Aminosäure steht und R" ein Alkylrest mit 1 - 10 C-Atomen ist.

6. Implantat nach Anspruch 5, bei dem R ein Substituent ist, der durch Kupplung mit einem Methyl- oder Ethylester der Aminosäuren Glycin, Alanin, Valin oder Phenylalanin entsteht.

7. Verwendung von biokorrodierbaren Polyphosphazenen als Beschichtungsmaterial für einen Stent aus einem biokorrodierbaren metallischen Werkstoff.
